# EUROPEAN PATENT SPECIFICATION

(11) **EP 1 233 760 B1**
(45) Date of publication and mention of the grant of the patent: **08.09.2004**
(21) Application number: 00976163.6
(22) Date of filing: 20.11.2000
(51) Int. Cl.: A61K 9/20, A61K 9/48

(54) **ORALLY ADMINISTRABLE PHARMACEUTICAL PRODUCT AND METHOD OF FABRICATION THEREOF**
ORAL ANZUWENDENDES PHARMAZEUTISCHES PRODUKT UND HERSTELLUNGSVERFAHREN
PRODUIT PHARMACEUTIQUE ADMINISTRABLE ORALEMENT ET PROCEDE DE FABRICATION CORRESPONDANT

(30) Priority: 03.12.1999 GB 9928511
(43) Date of publication of application: 28.08.2002
(73) Proprietor: pSiMedica Limited, Worcestershire, WR14 3SZ (GB)
(72) Inventor: CANHAM, Leigh Trevor, Malvern Worcestershire WR14 3PS (GB); WARD, Michael Charles Leslie, Malvern Worcestershire WR14 3PS (GB)
(74) Representative: Bowdery, Anthony Oliver
(86) International application number: PCT/GB2000/004378
(87) International publication number: WO 2001/039748

(56) References cited:
- WO-A-98/00107
- WO-A-99/09960
- WO-A-99/52590
- US-A- 5 792 048

## Description

This invention relates to orally administrable pharmaceutical products and to methods of fabrication for such products. More particularly the invention relates to an orally administrable pharmaceutical product that comprises information about the product, the information being protected by a material that is resistant to the gastric environment.

Drugs are frequently taken orally by a patient, the drug being absorbed by the digestive system of the patient. Orally administered pharmaceutical products include tablets, pellets, capsules, powders, suspensions and solutions. Capsules comprise a shell that contains the drug; the size and shape of the capsule being such that the patient can swallow the capsule. Capsules can be used to deliver beneficial substances other than drugs, such as nutrients and minerals. Usually the shell comprises gelatine and is formed from two halves, the halves being bonded together. The gelatine is rapidly disfigured in the gastric tract permitting the gastric fluids to penetrate the shell and reach the drug.

Tablets and pellets comprise a sample of solid material having a shape and size that allows a patient to swallow the tablet or pellet. Prior art tablets and pellets are typically prepared by compression or moulding of the solid material; the material may comprise a beneficial substance combined with one or more of: a diluent, a coating, a colorant, and a disintegrant. Diluents are fillers used in preparing tablets or pellets of the appropriate size and consistency; disintegrants are used for the break up and separation of the tablet's or pellet's compressed ingredients.

There are several different types of tablet and pellet coating. Excipient coatings are used to make the beneficial substance, or other material from which the tablet or pellet is comprised, more palatable to the patient. For example the beneficial substance may have an unpleasant or bitter taste which could be concealed by the coating.

There are a number of risks associated with drug administration. For example a patient may take a dosage that is in appropriate for her or his condition. A patient's condition may require the administration of a number of different pharmaceutical products at different times of the day; if the products are not clearly distinguished from each other then the patient may take the wrong product at the wrong time. Children may take a drug found in the home, mistaking it for candy or simply to emulate the patient for whom the drug has been prescribed. A patient or person associated with the patient may become depressed and deliberately take a large dose of a drug with the object of ending their life.

These risks can be reduced by appropriate packaging and labelling such as tamper-evident packaging, child-resistant safety containers, drug package inserts, and conspicuous labelling. Regulations continue to evolve so that even the drugs themselves often need individual labelling. According to FDA regulations effective from 1995 all solid dosage forms for human consumption must be imprinted with some form of product specific identification codes. Such imprints, together with the product's size shape and colour, enable the identification of a drug and its manufacturer prior to use.

Two established ways of enhancing tablet identification are: (a) polymer coating of intagliated tablet cores and (b) printing information relating to the tablet by offset or ink-jet technology. Typically orally administered drugs release their contents in the gastric tract of the patient. This means that a polymer coating will typically break up or becoming distorted in the gastric tract resulting in obliteration of information associated with the coating. Alternatively, if the information is printed onto the tablet, then the break up of the tablet in the gastric tract will also result in loss of the information.

When a patient has consumed a drug in a quantity or of a type that endangers the health of the patient, then such loss of information in the gastric environment may present a problem. It is essential that doctors treating such a patient be able to identify the drug as soon as possible so that remedial action can be taken. A rapid method of obtaining information about a pharmaceutical product would therefore be of great value in such an overdose situation.

The following prior art is of relevance to this application: WO 9909960 A, WO 9800107 A, US 5792048 A, US 5700998 A, and US 5079006 A. WO 9909960 relates to the use of sticking plaster as an information carrier. WO 9800107 relates to the use of microchips in the control of drug release. US 5792048 relates to the use of capsules that contains information relating to the identity of the person who has consumed the capsule. US 5,700,998 relates to the use of a code applied to the surface of capsules and tablets; the code is applied to ensure that the tablet or capsule is correctly administered. Finally, US 5079006 relates to the use of a magnetic material to detect whether a drug has entered the gastrointestinal environment.

It is an object of this invention to provide new pharmaceutical products that reduce the above mentioned problems. It is a further object of this invention to provide methods of fabricating such pharmaceutical products.

According to a first aspect, the invention provides an orally administrable pharmaceutical product comprising an information carrier having a form and composition such that information is recorded by the carrier; wherein the information carrier comprises a resistant material having a composition such that the resistant material is resistant to the gastric environment.

Preferably at least part of the resistant material is arranged such that, when the pharmaceutical product is located in a gastric tract, it forms a barrier that protects the recorded information from the gastric environment.

Advantageously the resistant material is shaped in such a manner that the information is recorded by the shape of the resistant material.

The information may be encoded, for example a colour code may be used in which a particular colour corresponds to a type of pharmaceutical product. Preferably the information comprises alphanumeric characters. Advantageously the orally administrable pharmaceutical product comprises at least one beneficial substance and the information relates at least partly to the or at least one of the beneficial substances, more advantageously the information comprises the identity of the or at least one of the beneficial substances. The identity of the beneficial substance may be encoded on the information carrier, for example the identity may be encoded in the form of a bar code.

The barrier may substantially surround the recorded information. Regardless of whether the resistant material surrounds the recorded information or is shaped to record the information, it forms a barrier to corrosion that tends to preserve the information.

Advantageously the information carrier comprises a semiconductor; more preferably the information carrier comprises silicon.

The information carrier may comprise a semiconductor chip or a silicon chip.

Advantageously the information is recorded by engraving the semiconductor chip to form characters on the chip. The characters may be engraved photolithographically.

By arranging the resistant material in such a way that it preserves the information in the stomach of the patient, the information remains intact. In the event that an overdose occurs, the patient's stomach may be pumped, and the contents of the stomach examined to locate the information carrier. Once located, the information present in the carrier will allow the most effective remedial action to be taken. The information, present on the carrier, may be read using a microscope.

Throughout this specification the term "patient" is to be taken to mean any animal or human to which a pharmaceutical product may be orally asdministered.

For the purposes of this specification the phrase: "resistant material that is resistant to the gastric environment" means that the resistant material is substantially uncorroded in simulated human gastric fluid when immersed for a period of less than 10 minutes. Preferably the resistant material has a composition such that it is substantially uncorroded when immersed in simulated human gastric fluid for a period of up to 1 hour. More preferably the resistant material has a composition such that it is substantially uncorroded when immersed in simulated human gastric fluid for a period of up to 10 hours.

It is advantageous to provide as much information about the composition of the pharmaceutical product as possible. If the product comprises a drug then information about the drug, the dosage of the drug, batch number, and its expiry date may be of value. It is also advantageous to provide this information in as compact a form as possible. For this reason the use of an information carrier comprising a semiconductor is advantageous since it is possible to engrave large quantities of information on small samples of semiconductor. Techniques that allow such compaction of information include electron beam lithography, laser ablation, photolithography, and other micromachining techniques. Silicon is a semiconductor that may be used to form at least part of the resistant material. Silicon has been shown to have high levels of resistance to gastric corrosion. Therefore information engraved onto silicon is resistant to gastric corrosion.

However, the information carrier need not comprise a semiconductor. The information carrier may comprise any material upon which information may be recorded, whether by printing, engraving, or by some other technique.

The largest dimension of the information carrier may be less than 10mm. Alternatively the largest dimension of the information carrier may be less than 2 mm. The dimensions of the information carrier may also be sufficiently small to have substantially no effect upon the delivery of the beneficial substance.

Advantageously the resistant material comprises a semiconductor. More advantageously the resistant material comprises silicon.

Preferably the information carrier comprises at least one micromachined component.

Advantageously the information is recorded onto the information carrier photolithographically. Preferably information is recorded onto the information carrier by electron beam lithography. The information may also be recorded onto the information carrier by laser ablation.

Advantageously the information carrier comprises a fluorescent or photoluminescent material; more advantageously the fluorescent or photoluminescent material comprises a fluorescent or photoluminescent semiconductor; yet more advantageously the semiconductor comprises porous silicon.

It is advantageous that the information carrier comprises a fluorescent or photoluminescent material because this assists location of the carrier in the contents of the gastrointestinal tract. Once the stomach of a patient has been pumped its contents may be illuminated with light of an appropriate wavelength, causing the information carrier to fluoresce, and facilitating identification.

Preferably the orally administrable product comprises a beneficial substance.

Throughout this specification the term "beneficial substance" means a substance that has an overall beneficial effect upon the patient when administered in an appropriate dose. The term "beneficial substance" includes drugs and minerals. The term "beneficial substance" also includes substances that may be, to some extent, toxic to the patient provided the overall effect upon the patient was beneficial. For example anti-cancer drugs are often, to some degree, toxic to the patient. For the avoidance of doubt the term "beneficial substance" further includes substances that are harmful to the patient when administered at an inappropriate dose.

Advantageously the orally administrable pharmaceutical product may comprise at least one moulded plastic component.

Preferably the recorded information comprises at least one character, the or at least one of the characters having a largest dimension less than 100µm; more preferably the largest dimension is less than 50µm; yet more preferably the largest dimension is less than 20µm.

Advantageously the information carrier may comprise a beneficial substance; more advantageously the information carrier comprises a beneficial substance together with porous and/or polycrystalline silicon, the arrangement of the beneficial substance being such that corrosion of the porous and or polycrystalline silicon in the gastrointestinal tract of the patient results in release of the beneficial substance.

The arrangement of a beneficial substance and a sample of porous and/or polycrystalline silicon in this manner is disclosed in GB 9924334.7.

Preferably the pharmaceutical product is a capsule and comprises a shell, the beneficial substance being disposed within the shell. More preferably the information carrier is disposed within the capsule shell.

Advantageously the information carrier is not located at the surface of the pharmaceutical product. More preferably the information carrier does not form part of the surface of the pharmaceutical product.

The information carrier may be located in the interior of the pharmaceutical product. The information carrier may be obscured by the material (not including the material that comprises the information carrier) comprising the pharmaceutical product.

According to a second aspect the invention provides a method of fabricating a pharmaceutical product comprising the step of recording information onto an information carrier; wherein the method further comprises the step of at least partly forming the information carrier from a resistant material that is resistant to the gastric environment.

Preferably the method of fabricating the pharmaceutical product further comprises the step of arranging the resistant material in such a manner that, when the information carrier is located in the gastric environment, the resistant material forms a barrier that protects the recorded information from the gastric environment.

Advantageously the recording step comprises the step of shaping at least part of the resistant material; more advantageously the step of shaping the resistant material comprises the step of engraving the resistant material.

Preferably the method of fabricating the pharmaceutical product comprises the further step of micromachining at least part of the material from which the information carrier if formed.

Advantageously the method of fabricating the pharmaceutical product comprises the step of photolithographically engraving at least part of the material from which the information carrier is formed.

Preferably method of fabricating the pharmaceutical product comprises the step of engraving, by electron beam lithography, at least part of the material from which the information carrier is formed.

Alternatively the method of fabricating the pharmaceutical product comprises the step of engraving, by laser ablation, at least part of the material from which the information carrier is formed.

Advantageously the information carrier comprises a plastic material and the step of recording information onto an information carrier comprises the step of moulding the plastic material; more advantageously the step of moulding the plastic material comprises the step of at least partly filling a semiconductor mould with the plastic material; yet more advantageously the semiconductor mould comprises silicon.

Preferably the semiconductor is micromachined to form the mould.

Advantageously the method of fabricating a pharmaceutical product further comprises the step of combining a beneficial substance with the information carrier; more advantageously the recording step is performed in such a manner that the recorded information relates at least partly to the beneficial substance.

According to a third aspect, the invention provides an information carrier comprising a bulk crystalline semiconductor region and porous semiconductor region wherein the information carrier is shaped in such a manner that information is recorded by the shape of the information carrier.

The largest dimension of the bulk crystalline region may be less than 10mm. Alternatively the largest dimension of the bulk crystalline region may be less than 2 mm.

Preferably the recorded information comprises at least one character, the or at least one of the characters having a largest dimension less than 100µm; more preferably the largest dimension is less than 50µm; yet more preferably the largest dimension is less than 20µm.

The bulk and porous semiconductor regions may comprise different semiconductors; more preferably both the bulk and porous regions comprise silicon.

The bulk semiconductor region may be shaped in such a manner that the information is recorded by the bulk region. Alternatively the porous region may be shaped in such a manner that the information is recorded by the porous region.

According to a fourth aspect the invention provides a product comprising an information carrier, the information carrier comprising a bulk crystalline semiconductor region and a porous semiconductor region; wherein the information carrier is shaped in such a manner that information is recorded by the shape of the information carrier.

The product may be a pharmaceutical product or it may be some other product. The information carrier may be used as a covert marker to identify the product. For example the information carrier may be used to identify the product as originating from a particular manufacturer.

According to a fifth aspect the invention provides a method of fabricating an information carrier comprising a bulk semiconductor region and a porous semiconductor region, the method comprising the steps of micromachining the bulk semiconductor and/or the porous semiconductor in such a manner that information is recorded by the micromachined bulk and/or porous semiconductor.

Preferably the method of fabricating the information carrier further comprises the step of taking a sample bulk silicon and anodising it to form porous silicon.

In order that the invention might be more fully understood, embodiments thereof will now be described, by way of example only, with reference to accompanying drawings, in which:
- Figure 1: is an illustration of a pharmaceutical product, comprising an information carrier, according to the invention;
- Figure 2: is a schematic diagram of processing steps, according to the invention, required to fabricate the information carrier shown in figure 1;
- Figure 3: shows the results of an experiment showing the effect of immersing a sample of bulk crystalline silicon in simulated human gastric fluid; and
- Figure 4: shows the pharmaceutical product of figure 1, located in a gastric environment.

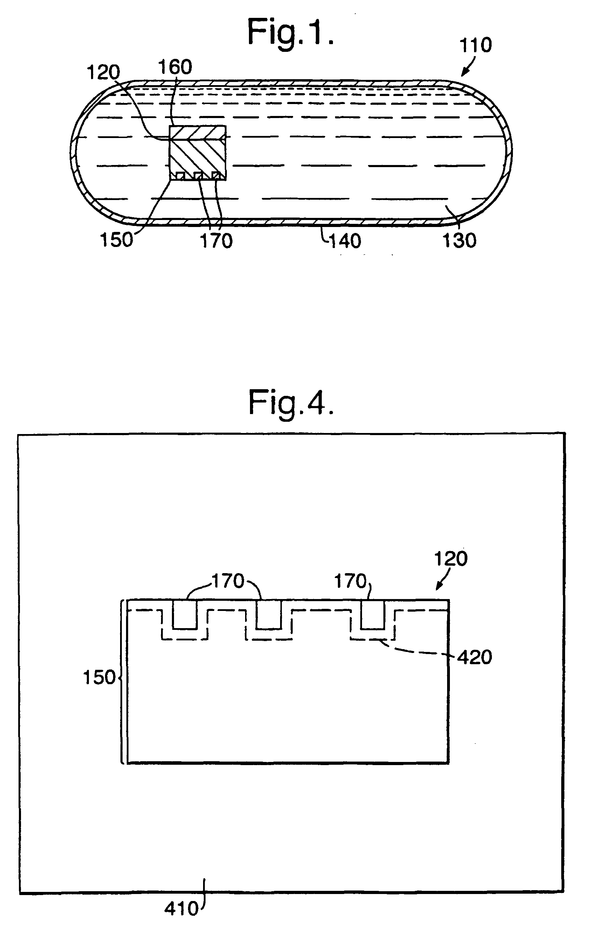

Figure 1 shows a schematic cross section of an orally administrable pharmaceutical product, according to the invention, in the form of a capsule 110. The capsule 110 comprises an information carrier in the form of a silicon chip 120, a beneficial substance 130, and a gelatin shell 140. The silicon chip 120 has a bulk crystalline silicon region 150 and a porous silicon region 160. Recorded information in the form of a number of alphanumeric characters 170 are engraved into the bulk crystalline silicon region 150. The characters 170 may relate to the identity of the beneficial substance 130, to the manufacturer of the beneficial substance 130, and to the dose of the beneficial substance 130 contained in the capsule 110.

When the capsule 110 is consumed by a patient the shell 140 becomes distorted in the patient's stomach releasing the beneficial substance 130. In the event that the patient has taken an overdose, in other words a number of capsules 110, the stomach may be pumped to empty it of its contents. The stomach contents, containing the silicon chips 120, are then illuminated with light having a wavelength between 300 and 500 nm. The porous silicon 160, associated with each pharmaceutical product, fluoresces at wavelengths between 400 and 900 nm. The fluorescence of the porous silicon 160 allows the silicon chips 120 to be located in the stomach contents. Once a silicon chip 120 has been located it is removed from the stomach contents, cleaned and placed under a microscope. The characters 170 are read and appropriate remedial action is taken once the identity and dose of the beneficial substance 130 have been identified. The dose may easily be determined by counting the number of fluorescing silicon chips 120 and combining this information with information contained on each chip 120.

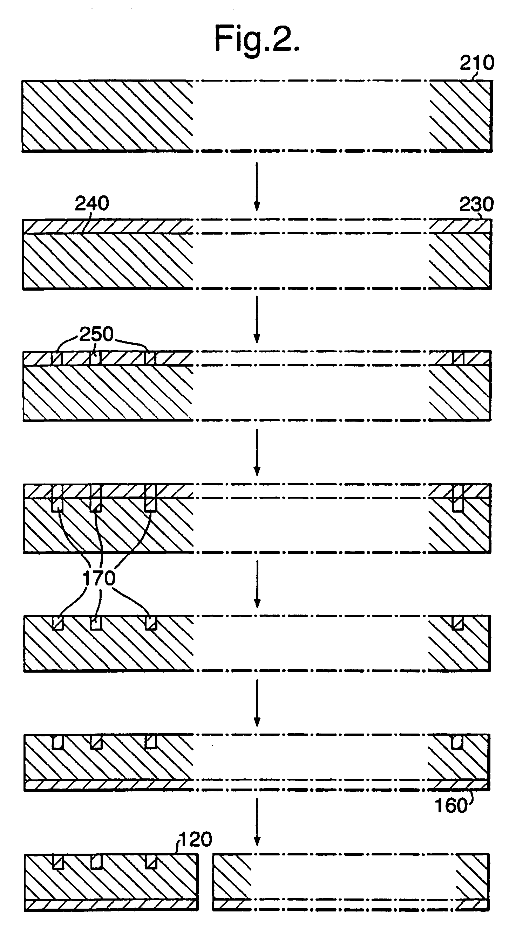

Figures 2 shows the processing steps involved in fabricating a number of silicon chips 120, from a silicon wafer 210 that comprises bulk crystalline silicon. A 1 µm thick silicon nitride layer 230 is deposited onto one of the surfaces 240 of the silicon wafer 210. The silicon nitride, once patterned, provides a stencil for isotropic etching of characters 170 into the wafer 210. An organic photosensitive resist is deposited or spun onto the silicon nitride layer 230, photolithographically exposed and developed. The wafer 210 is then reactively ion etched to provide etch windows such as 250 through the silicon nitride layer 230. The organic resist is then stripped off using oxygen plasma ashing or acetone. The wafer 210 is then immersed in a mixture of potassium hydroxide solution and ethanol to isotropically etch a first set of characters 170 into the wafer 210. The potassium hydroxide etched wafer 210 is then exposed to ion milling to remove remnants of the silicon nitride layer 230. The wafer 210 is then anodised in aqueous HF, as described in US Patent No. 5,348,618, to form a layer 160 of porous silicon. The porosity of the layer may be in the range 50% to 90%, and the thickness of the layer may be in the range 100 nm to 100µm. Finally the patterned wafer is diced to yield a number of chips 120 on which characters 170 have been etched. Only one porous layer 160 is shown in figure 2. However, it is possible to anodise both surfaces of the wafer 210 so that chips 120 having porous silicon on two surfaces result. It may be advantageous to have two such layers of porous silicon since this may assist in differentiating the chip from the stomach contents. In other words the existence of two porous surfaces reduces the chances of the fluorescent porous silicon being concealed from the ultraviolet radiation used to detect the chip 120.

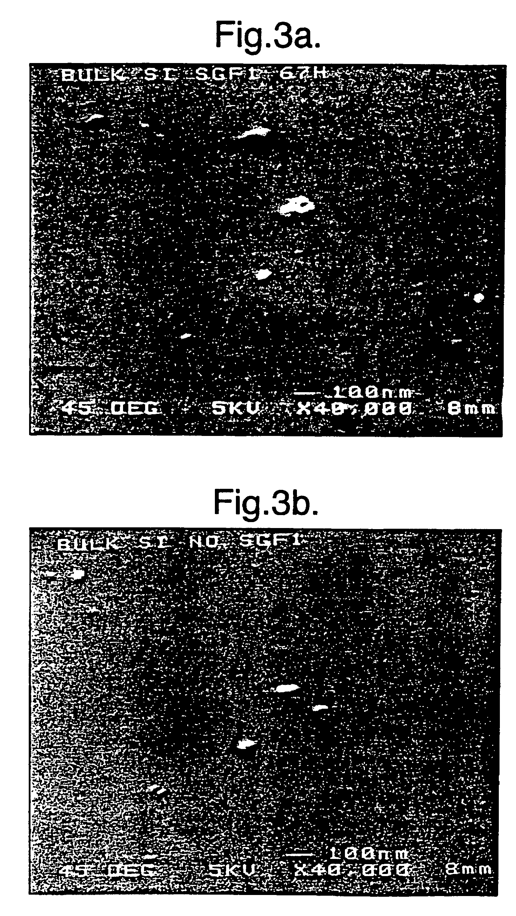

Bulk crystalline samples of silicon were tested in both simulated gastric fluid following the formulations of Cooper et al (Hydrodysis Studies on oleamide in simulated GI fluids, Food Additives + Contaminants 12(6) p769-777 (1995)). The simulated gastric fluid (SGF) was prepared by first diluting 1M HCl to 0.07M and volume 500cm³. 1.6g of pepsin and 1g of NaCl was then added and the solution incubated at 37°C. References to simulated gastric fluid, made in any part of this specification, refer to SGF having this composition and prepared in this manner. A silicon wafer were immersed in SGF for a period of 67 hours. Figure 3 shows the surface of a silicon wafer after (figure 3a) and before (figure 3b) 67 hours of immersion in SGF; as can be seen there is no appreciable corrosion of the wafer surface (the slight differences between the two images are due to organic deposition. It therefore follows that bulk crystalline silicon is resistant to the gastric environment.

Figure 4 shows the chip 120, comprising a bulk crystalline region 150, located in a gastric environment 410. Part of the bulk crystalline region 150 forms a barrier 420 that protects characters 170 from the gastric environment; the barrier comprising the same material and being integral with the rest of the region 150. The characters 170 are recorded by the shape of the resistant material, in this case bulk crystalline silicon.

An orally administrable pharmaceutical product according to the invention need not comprise an information carrier in which the information is recorded in a material that is resistant to the gastric environment. The information may be protected from the gastric environment, once the pharmaceutical product is located in the stomach, by a barrier formed from a resistant material. Such a barrier may surround the recorded information and, if the information is to be viewed, it may also be transparent.

## Claims

1. An orally administrable pharmaceutical product comprising an information carrier having a form and composition such that information is recorded by the carrier; wherein the information carrier comprises a resistant material that is resistant to the gastric environment.

2. A pharmaceutical product according to Claim 1 wherein the at least part of the resistant material is arranged such that, when the pharmaceutical product is located in a gastric tract, it forms a barrier that protects the recorded information from the gastric environment.

3. A pharmaceutical product according to Claim 1 wherein the resistant material is shaped in such a manner that the information is recorded by the shape of the resistant material.

4. A pharmaceutical product according to Claim 1 wherein the largest dimension of the information carrier is less than 10 mm.

5. A pharmaceutical product according to Claim 3 wherein the information is engraved into the resistant material.

6. A pharmaceutical product according to Claim 1 wherein the information carrier comprises a semiconductor.

7. A pharmaceutical product according to Claim 1 wherein the resistant material comprises a semiconductor.

8. A pharmaceutical product according to either Claim 6 or Claim 7 wherein the semiconductor comprises silicon.

9. A pharmaceutical product according to Claim 1 wherein the information carrier comprises a fluorescent or photoluminescent material.

10. A pharmaceutical product according to Claim 9 wherein the fluorescent or photoluminescent material comprises porous silicon.

11. A pharmaceutical product according to Claim 1 wherein the information carrier comprises at least one micromachined component.

12. A pharmaceutical product according to Claim 1 wherein the product comprises at least one moulded plastic component.

13. A pharmaceutical product according to Claim 1 wherein the product further comprises a beneficial substance and wherein the recorded information relates at least partly to the beneficial substance.

14. A pharmaceutical product according to Claim 1 wherein the recorded information comprises at least one character, the or at least one of the characters having a largest dimension less than 100µm.

15. A method of fabricating a pharmaceutical product comprising the step of recording information onto an information carrier; wherein the method further comprises the step of at least partly forming the information carrier from a resistant material that is resistant to the gastric environment.

16. A method according to Claim 15 wherein the method further comprises the step of arranging the resistant material in such a manner that, when the information carrier is located in the gastric environment, the resistant material forms a barrier that protects the recorded information from the gastric environment.

17. A method according to Claim 15 wherein the recording step comprises the step of shaping at least part of the resistant material.

18. A method according to Claim 17 wherein the step of shaping the resistant material comprises the step of engraving the resistant material.

19. A method according to Claim 15 wherein the method comprises the further step of micromachining at least part of the material from which the information carrier if formed.

20. A method according to Claim 15 wherein the method comprises the step of photolithographically engraving at least part of the material from which the information carrier is formed.

21. A method according to Claim 15 wherein the method comprises the step of engraving, by electron beam lithography, at least part of the material from which the information carrier is formed.

22. A method according to Claim 15 wherein the information carrier comprises a plastic material and wherein the step of recording information onto an information carrier comprises the step of moulding the plastic material.

23. A method according to Claim 22 wherein the step of moulding the plastic material comprises the step of at least partly filling a semiconductor mould with the plastic material.

24. A method according to Claim 23 wherein the semiconductor mould comprises silicon.

25. A method according to Claim 23 wherein the method further comprises the step of micromachining the semiconductor to form the mould.

26. A method according to Claim 15 wherein the method further comprises the step of combining a beneficial substance with the information carrier and wherein the recording step is performed in such a manner that the recorded information relates at least partly to the beneficial substance.

27. An information carrier for use in a pharmaceutical product comprising a bulk crystalline semiconductor region and porous semiconductor region wherein the information carrier is shaped in such a manner that information is recorded by the shape of the information carrier and wherein the information relates to a beneficial substance.

28. An information carrier according to Claim 27 wherein the bulk and porous regions comprise different semiconductors.

29. An information carrier according to Claim 27 wherein both the bulk and porous regions comprise silicon.

30. An information carrier according to Claim 27 wherein the bulk region is shaped in such a manner that the information is recorded by the bulk region.

31. An information carrier according to Claim 27 wherein the porous region is shaped in such a manner that the information is recorded by the porous region.

32. An information carrier according to Claim 27 wherein the largest dimension of the information carrier is less than 10 mm.

33. An information carrier according to Claim 27 wherein the recorded information comprises at least one character, the or at least one of the characters having a largest dimension less than 100µm.

34. A method of fabricating an information carrier comprising a bulk semiconductor region and a porous semiconductor region, the method comprising the steps of micromachining the bulk semiconductor and/or the porous semiconductor in such a manner that information is recorded by the shape of the bulk semiconductor and/or porous semiconductor.

35. A method according to Claim 34 wherein the method further comprises the step of taking a sample bulk silicon and anodising it to form porous silicon.

36. A method according to Claim 34 wherein the micromachining step is performed in such a manner that the largest dimension of the information carrier is less than 10 mm.

37. A method according to Claim 36 wherein the largest dimension of the information carrier is less than 2 mm.

38. A method of fabricating an information carrier according to Claim 34 wherein the information comprises at least one character and wherein the micromachining step is performed in such a manner that the or at least one of the characters has a largest dimension less than 100µm.

39. A method according to Claim 38 wherein the largest dimension of the character is less than 50µm.

40. A method of fabricating a product comprising an information carrier the method comprising the step of fabricating an information carrier by the method of Claim 34 wherein the micromachining is performed in such a manner that information relates to the properties of the product.

41. The use of an information carrier as defined in any one of Claims 27 to 33 in the preparation of a medicament for use in any surgical, therapeutic or diagnostic method practised on a human or animal patient.

## Patentansprüche

1. Oral verabreichbares pharmazeutisches Produkt, das einen Informationsträger einer solchen Form und Zusammensetzung aufweist, dass Information durch den Träger aufgezeichnet ist, wobei der Informationsträger ein beständiges Material umfasst, das gegen die Umgebung im Magen beständig ist.

2. Pharmazeutisches Produkt nach Anspruch 1, bei dem mindestens ein Teil des beständigen Materials so vorgesehen ist, dass das pharmazeutische Produkt, wenn es sich in einem Magentrakt befindet, eine Barriere bildet, welche die aufgezeichnete Information vor der Umgebung im Magen schützt.

3. Pharmazeutisches Produkt nach Anspruch 1, bei dem das beständige Material so geformt ist, dass die Information durch die Form des beständigen Materials aufgezeichnet ist.

4. Pharmazeutisches Produkt nach Anspruch 1, bei dem die größte Abmessung des Informationsträgers weniger als 10 mm beträgt.

5. Pharmazeutisches Produkt nach Anspruch 3, bei dem die Information in das beständige Material eingraviert ist.

6. Pharmazeutisches Produkt nach Anspruch 1, bei dem der Informationsträger einen Halbleiter umfasst.

7. Pharmazeutisches Produkt nach Anspruch 1, bei dem das beständige Material einen Halbleiter umfasst.

8. Pharmazeutisches Produkt nach Anspruch 6 oder 7, bei dem der Halbleiter Silicium ist.

9. Pharmazeutisches Produkt nach Anspruch 1, bei dem der Informationsträger ein fluoreszierendes oder photolumineszierendes Material umfasst.

10. Pharmazeutisches Produkt nach Anspruch 9, bei dem das fluoreszierende oder photolumineszierende Material poröses Silicium umfasst.

11. Pharmazeutisches Produkt nach Anspruch 1, bei dem der Informationsträger mindestens einen mikrobearbeiteten Bestandteil aufweist.

12. Pharmazeutisches Produkt nach Anspruch 1, das mindestens einen geformten Kunststoffbestandteil aufweist.

13. Pharmazeutisches Produkt nach Anspruch 1, das ferner eine nützliche Substanz enthält und bei dem sich die aufgezeichnete Information zumindest teilweise auf die nützliche Substanz bezieht.

14. Pharmazeutisches Produkt nach Anspruch 1, bei dem die aufgezeichnete Information mindestens ein Zeichen umfasst, wobei das oder mindestens eines der Zeichen eine größte Abmessung aufweist, die kleiner als 100 µm ist.

15. Verfahren zur Herstellung eines pharmazeutischen Produkts, das den Schritt der Aufzeichnung von Information auf einem Informationsträger umfasst, wobei das Verfahren ferner den Schritt der zumindest teilweisen Erzeugung des Informationsträgers aus einem beständigen Material umfasst, das gegen die Umgebung im Magen beständig ist.

16. Verfahren nach Anspruch 15, das ferner den Schritt des Vorsehens des beständigen Materials in der Weise umfasst, dass das beständige Material, wenn sich der Informationsträger in der Umgebung im Magen befindet, eine Barriere bildet, welche die aufgezeichnete Information vor der Umgebung im Magen schützt.

17. Verfahren nach Anspruch 15, bei dem der Schritt der Aufzeichnung den Schritt der Formgebung mindestens eines Teils des beständigen Materials umfasst.

18. Verfahren nach Anspruch 17, bei dem der Schritt der Formgebung des beständigen Materials den Schritt der Gravierung des beständigen Materials umfasst.

19. Verfahren nach Anspruch 15, das ferner den Schritt der Mikrobearbeitung mindestens eines Teils des Materials umfasst, aus dem der Informationsträger erzeugt wird.

20. Verfahren nach Anspruch 15, das den Schritt der photolithographischen Gravierung mindestens eines Teils des Materials umfasst, aus dem der Informationsträger erzeugt wird.

21. Verfahren nach Anspruch 15, das den Schritt der Gravierung mindestens eines Teils des Materials, aus dem der Informationsträger erzeugt wird, durch Elektronenstrahllithographie umfasst.

22. Verfahren nach Anspruch 15, bei dem der Informationsträger ein Kunststoffmaterial umfasst und der Schritt der Aufzeichnung von Information auf einem Informationsträger den Schritt der Formgebung des Kunststoffmaterials umfasst.

23. Verfahren nach Anspruch 22, bei dem der Schritt der Formgebung des Kunststoffmaterials den Schritt des mindestens teilweisen Füllens einer Halbleiterform mit dem Kunststoffmaterial umfasst.

24. Verfahren nach Anspruch 23, bei dem die Halbleiterform aus Silicium besteht.

25. Verfahren nach Anspruch 23, das ferner den Schritt der Mikrobearbeitung des Halbleiters zur Erzeugung der Form umfasst.

26. Verfahren nach Anspruch 15, das ferner den Schritt der Kombination einer nützlichen Substanz mit dem Informationsträger umfasst, wobei der Aufzeichnungsschritt so durchgeführt wird, dass sich die aufgezeichnete Information zumindest teilweise auf die nützliche Substanz bezieht.

27. Informationsträger zur Verwendung in einem pharmazeutischen Produkt, der einen kristallinen Bulk-Halbleiterbereich und einen porösen Halbleiterbereich aufweist, wobei der Informationsträger so geformt ist, dass Information durch die Form des Informationsträgers aufgezeichnet ist, und wobei sich die Information auf eine nützliche Substanz bezieht.

28. Informationsträger nach Anspruch 27, bei dem der Bulk-Bereich und der poröse Bereich unterschiedliche Halbleiter aufweisen.

29. Informationsträger nach Anspruch 27, bei dem sowohl der Bulk-Bereich als auch der poröse Bereich aus Silicium bestehen.

30. Informationsträger nach Anspruch 27, bei dem der Bulk-Bereich so geformt ist, dass die Information durch den Bulk-Bereich aufgezeichnet ist.

31. Informationsträger nach Anspruch 27, bei dem der poröse Bereich so geformt ist, dass die Information durch den porösen Bereich aufgezeichnet ist.

32. Informationsträger nach Anspruch 27, bei dem die größte Abmessung des Informationsträgers weniger als 10 mm beträgt.

33. Informationsträger nach Anspruch 27, bei dem die aufgezeichnete Information mindestens ein Zeichen umfasst, wobei das oder mindestens eines der Zeichen eine größte Abmessung von weniger all 100 µm aufweist.

34. Verfahren zur Herstellung eines Informationsträgers, der einen Bulk-Halbleiterbereich und eine porösen Halbleiterbereich aufweist, wobei das Verfahren die Schritte der Mikrobearbeitung des Bulk-Halbleiters und/oder des porösen Halbleiters in der Weise umfasst, dass Information durch die Form des Bulk-Halbleiters und/oder des porösen Halbleiters aufgezeichnet wird.

35. Verfahren nach Anspruch 34, das ferner den Schritt umfasst, in dem eine Probe von Bulk-Silicium genommen und unter Erzeugung von porösem Silicium anodisiert wird.

36. Verfahren nach Anspruch 34, bei dem der Schritt der Mikrobearbeitung so durchgeführt wird, dass die größte Abmessung des Informationsträgers weniger als 10 mm beträgt.

37. Verfahren nach Anspruch 36, bei dem die größte Abmessung des Informationsträgers weniger als 2 mm beträgt.

38. Verfahren zur Herstellung eines Informationsträgers nach Anspruch 34, wobei die Information mindestens ein Zeichen umfasst und der Schritt der Mikrobearbeitung so durchgeführt wird, dass das oder mindestens eines der Zeichen eine größte Abmessung von weniger als 100 µm aufweist.

39. Verfahren nach Anspruch 38, wobei die größte Abmessung des Zeichens weniger als 50 µm beträgt.

40. Verfahren zur Herstellung eines Produkts, das einen Informationsträger aufweist, wobei das Verfahren den Schritt der Herstellung eines Informationsträgers nach dem Verfahren von Anspruch 34 umfasst, wobei die Mikrobearbeitung so durchgeführt wird, dass sich die Information auf die Eigenschaften des Produkts bezieht.

41. Verwendung eines Informationsträgers nach einem der Ansprüche 27 bis 33 bei der Herstellung eines Arzneimittels zur Verwendung in einem chirurgischen, therapeutischen oder diagnostischen Verfahren, das an einem menschlichen oder tierischen Patienten vorgenommen wird.

## Revendications

1. Produit pharmaceutique administrable oralement, comprenant un vecteur d'informations de forme et de composition telles que l'information soit enregistrée par le vecteur, le vecteur d'informations comprenant un matériau résistant qui résiste à l'environnement gastrique.

2. Produit pharmaceutique selon la revendication 1, dans lequel au moins une partie du matériau résistant soit disposée de telle sorte que, lorsque le produit pharmaceutique est localisé dans les voies gastriques, il forme une barrière qui protège l'information enregistrée de l'environnement gastrique.

3. Produit pharmaceutique selon la revendication 1, dans lequel le matériau résistant est formé de telle sorte que l'information soit enregistrée par la forme du matériau résistant.

4. Produit pharmaceutique selon la revendication 1, dans lequel la dimension maximale du vecteur d'informations est inférieure à 10 mm.

5. Produit pharmaceutique selon la revendication 3, dans lequel l'information est gravée dans le matériau résistant.

6. Produit pharmaceutique selon la revendication 1, dans lequel le vecteur d'informations comprend un semi-conducteur.

7. Produit pharmaceutique selon la revendication 1, dans lequel le matériau résistant comprend un semi-conducteur.

8. Produit pharmaceutique selon la revendication 6 ou la revendication 7, dans lequel le semi-conducteur comprend du silicium.

9. Produit pharmaceutique selon la revendication 1, dans lequel le vecteur d'informations comprend un matériau fluorescent ou photo-luminescent.

10. Produit pharmaceutique selon la revendication 9, dans lequel le matériau fluorescent ou photo-luminescent comprend du silicium poreux.

11. Produit pharmaceutique selon la revendication 1, dans lequel le vecteur d'informations comprend au moins un composé micro-usiné.

12. Produit pharmaceutique selon la revendication 1, dans lequel le produit comprend au moins un composant en plastique moulé.

13. Produit pharmaceutique selon la revendication 1, dans lequel le produit comprend en outre une substance bénéfique et dans lequel, l'information enregistrée concerne au moins en partie la substance bénéfique.

14. Produit pharmaceutique selon la revendication 1, dans lequel l'information enregistrée comprend au moins un caractère, les caractères ou au moins l'un d'entre eux ayant une dimension maximale inférieure à 100 µm.

15. Procédé de fabrication d'un produit pharmaceutique comprenant l'étape consistant à enregistrer des informations sur un vecteur d'informations, dans lequel le procédé comprend en outre l'étape consistant à former au moins partiellement le vecteur d'informations à partir d'un matériau résistant qui résiste à l'environnement gastrique.

16. Procédé selon la revendication 15, dans lequel le procédé comprend en outre l'étape consistant à disposer le matériau résistant de telle sorte que, lorsque le vecteur d'informations est localisé dans l'environnement gastrique, le matériau résistant forme une barrière qui protège l'information enregistrée de l'environnement gastrique.

17. procédé selon la revendication 15, dans lequel l'étape d'enregistrement comprend l'étape consistant à former au moins partiellement le matériau résistant.

18. Procédé selon la revendication 17, dans lequel l'étape de formation du matériau résistant comprend l'étape consistant à graver le matériau résistant.

19. Procédé selon la revendication 15, dans lequel le procédé comprend l'étape ultérieure de micro-usinage d'au moins une partie du matériau à partir duquel le vecteur d'informations est formé.

20. Procédé selon la revendication 15, dans lequel le procédé comprend l'étape constituant à graver par photolithographie, au moins une partie du matériau à partir duquel le vecteur d'informations est formé.

21. Procédé selon la revendication 15, dans lequel le procédé comprend l'étape constituant à graver, par lithographie par faisceaux d'électrons, au moins une partie du matériau à partir duquel le vecteur d'informations est formé.

22. Procédé selon la revendication 15, dans lequel le vecteur d'informations comprend un matériau plastique et dans lequel l'étape d'enregistrement de l'information sur un vecteur d'informations comprend l'étape consistant à mouler le matériau plastique.

23. Procédé selon la revendication 22, dans lequel l'étape de moulage du matériau plastique comprend l'étape consistant à remplir au moins partiellement le moule semi-conducteur avec le matériau plastique.

24. Procédé selon la revendication 23, dans lequel le moule semi-conducteur comprend du silicium.

25. Procédé selon la revendication 23, dans lequel le procédé comprend en outre l'étape consistant à micro-usiner le semi-conducteur pour former le moule.

26. Procédé selon la revendication 15, dans lequel le procédé comprend en outre l'étape consistant à combiner une substance bénéfique avec le vecteur d'informations et dans lequel l'étape d'enregistrement est réalisée de telle sorte que l'information enregistrée concerne au moins partiellement la substance bénéfique.

27. Vecteur d'informations destiné à l'utilisation dans un produit pharmaceutique comprenant une région semi-conductrice cristalline en masse et une région semi-conductrice poreuse, le vecteur d'informations étant formé de telle sorte que l'information soit enregistrée par la forme du vecteur d'informations et l'information concernant une substance bénéfique.

28. Vecteur d'informations selon la revendication 27, dans lequel les régions en masse et poreuses comprennent différents semi-conducteurs.

29. Vecteur d'informations selon la revendication 27, dans lequel les régions en masse et poreuses comprennent toutes deux du silicium.

30. Vecteur d'informations selon la revendication 27, dans lequel la région en masse est formée de telle sorte que l'information soit enregistrée par la région en masse.

31. Vecteur d'informations selon la revendication 27, dans lequel la région poreuse est formée de telle sorte que l'information soit enregistrée par la région poreuse.

32. Vecteur d'informations selon la revendication 27, dans lequel la dimension maximale du vecteur d'informations est inférieure à 10 mm.

33. Vecteur d'informations selon la revendication 27, dans lequel l'information enregistrée comprend au moins un caractère, les caractères ou au moins l'un d'entre eux ayant une dimension maximale inférieure à 100 µm.

34. Procédé de fabrication d'un vecteur d'informations comprenant une région semi-conductrice en masse et une région semi-conductrice poreuse, le procédé comprenant les étapes de micro-usinage du semi-conducteur en masse et/ou du semi-conducteur poreux de telle manière que l'information soit enregistrée par la forme du semi-conducteur en masse et/ou du semi-conducteur poreux.

35. Procédé selon la revendication 34, dans lequel le procédé comprend en outre l'étape consistant à prélever un échantillon de silicium en masse et à l'anodiser pour former du silicium poreux.

36. Procédé selon la revendication 34, dans lequel l'étape de micro-usinage est réalisée de telle sorte que la dimension maximale du vecteur d'informations soit inférieure à 10 mm.

37. Procédé selon la revendication 36, dans lequel la dimension maximale du vecteur d'informations est inférieure à 2 mm.

38. Procédé de fabrication d'un vecteur d'informations selon la revendication 34, dans lequel l'information comprend au moins un caractère et dans lequel l'étape de micro-usinage est réalisée de telle sorte que les caractères ou au moins l'un d'entre eux présentent une dimension maximale inférieure à 100 µm.

39. Procédé selon la revendication 38, dans lequel la dimension maximale du caractère est inférieure à 50 µm.

40. Procédé de fabrication d'un produit comprenant un vecteur d'informations, le procédé comprenant l'étape de fabrication d'un vecteur d'informations par le procédé de la revendication 34, dans lequel le micro-usinage est réalisé de telle sorte que l'information concerne les propriétés du produit.

41. Utilisation d'un vecteur d'informations tel que défini dans l'une quelconque des revendications 27 à 33, dans la préparation d'un médicament utilisé dans tout procédé chirurgical, thérapeutique ou diagnostic mis en oeuvre sur un patient humain ou un animal.
